# EUROPEAN PATENT SPECIFICATION

(11) **EP 2 306 942 B1**
(45) Date of publication and mention of the grant of the patent: **16.05.2012**
(21) Application number: 09739820.0
(22) Date of filing: 30.04.2009
(51) Int. Cl.: A61F 5/00

(54) **GASTRIC VOLUME REDUCTION USING ANTERIOR TO POSTERIOR WALL JUNCTIONS**
REDUZIERUNG DES MAGENVOLUMENS MIT ANTERIOR/POSTERIOR-WANDVERBINDUNGEN
RÉDUCTION DE VOLUME GASTRIQUE AU MOYEN DE JONCTIONS DE PAROIS ANTÉRIEURE ET POSTÉRIEURE

(30) Priority: 01.05.2008 US 113686
(43) Date of publication of application: 13.04.2011
(73) Proprietor: Ethicon Endo-Surgery, Inc., Cincinnati, OH 45242 (US)
(72) Inventor: HARRIS, Jason, L., Mason OH 45040 (US); ZEINER, Mark, S., Mason OH 45040 (US); ALBRECHT, Thomas, E., Cincinnati OH 45242 (US); STOKES, Michael, J., Cincinnati OH 45244 (US); ALESI, Daniel, E., Lebanon OH 45036 (US); CRAINICH, Lawrence, Charlestown NH 03603 (US); ORTIZ, Mark, S., Milford OH 45150 (US)
(74) Representative: Tunstall, Christopher Stephen
(86) International application number: PCT/US2009/042337
(87) International publication number: WO 2009/135018

(56) References cited:
- EP-A- 1 749 506
- WO-A-2005/092210
- US-A- 4 458 681
- US-A- 4 558 699
- US-A1- 2006 195 139
- US-A1- 2007 021 761

## Description

### BACKGROUND OF THE INVENTION

### 1. Field of the Invention

The invention relates to bariatric surgery. More particularly, the invention relates to the creation of anterior and posterior wall junctions in reducing the effective volume of the gastric cavity.

### 2. Description of the Related Art

Obesity is a medical condition affecting more than 30% of the population in the United States. Obesity affects an individual's personal quality of life and contributes significantly to morbidity and mortality. Obese patients, i.e., individuals having a body mass index ("BMI") greater than 30, often have a high risk of associated health problems (e.g., diabetes, hypertension and respiratory insufficiency), including early death. With this in mind, and as those skilled in the art will certainly appreciate, the monetary and physical costs associated with obesity are substantial. In fact, it is estimated the costs relating to obesity are in excess of 100 billion dollars in the United States alone. Studies have shown that conservative treatment with diet and exercise alone may be ineffective for reducing excess body weight in many patients. Bariatrics is the branch of medicine that deals with the control and treatment of obesity. A variety of surgical procedures have been developed within the bariatrics field to treat obesity. The most common currently performed procedure is the Roux-en-Y gastric bypass (RYGB). This procedure is highly complex and is commonly utilized to treat people exhibiting morbid obesity. In a RYGB procedure a small stomach pouch is separated from the remainder of the gastric cavity and attached to a resectioned portion of the small intestine. This resectioned portion of the small intestine is connected between the "smaller" gastric cavity and a distal section of small intestine allowing the passage of food therebetween. The conventional RYGB procedure requires a great deal of operative time. Because of the degree of invasiveness, post-operative recovery can be quite lengthy and painful. Still more than 100,000 RYGB procedures are performed annually in the United States alone, costing significant health care dollars.

In view of the highly invasive nature of the RYGB procedure, other less invasive procedures have been developed. These procedures include gastric banding, which constricts the stomach to form an hourglass shape. This procedure restricts the amount of food that passes from one section of the stomach to the next, thereby inducing a feeling of satiety. A band is placed around the stomach near the junction of the stomach and esophagus. The small upper stomach pouch is filled quickly, and slowly empties through the narrow outlet to produce the feeling of satiety. Other forms of bariatric surgery that have been developed to treat obesity include Fobi pouch, bilio-pancreatic diversion and gastroplasty or "stomach stapling".

Morbid obesity is defined as being greater than 45.4 Kg (100 pounds) over one's ideal body weight. For individuals in this category, RYGB, gastric banding or another of the more complex procedures may be the recommended course of treatment due to the significant health problems and mortality risks facing the individual. However, there is a growing segment of the population in the United States and elsewhere who are overweight without being considered morbidly obese. These persons may be 9.1 to 13.6kg (20-30 pounds)overweight and want to lose the weight, but have not been able to succeed through diet and exercise alone. For these individuals, the risks associated with the RYGB or other complex procedures often outweigh the potential health benefits and costs. Accordingly, treatment options should involve a less invasive, lower cost solution for weight loss.

It is known to create cavity wall plications through endoscopic only procedures. However, operating solely within the interior of the gastric cavity limits the plication depth that can be achieved without cutting. Furthermore, access and visibility within the gastric and peritoneal cavities is limited in a purely endoscopic procedure as the extent of the reduction increases.

With the foregoing in mind, it is desirable to provides surgical weight loss restriction system that is inexpensive, with few potential complications, and that provide patients with a weight loss benefit while buying time for the lifestyle changes necessary to maintain the weight loss. The present invention provides such a restriction system

US 2006/0195139 A1 discloses methods and devices for externally creating a restriction on the stomach. The devices may be contoured to fit the stomach and can be anchored to the stomach.

US 4,458,681 discloses a stomach clamp with partially occluding arms that extend across a stomach. The clamp is clamped in place by bolting together opposed arm ends. the clamp is semi-permanently installed without penetration of stomach lumen or gastrointestinal tract. Engagement of the arms is reinforced with sutures through outer layers of the stomach.

US 4,558,699 discloses apparatus for restricting the passage of food through the stomach. The apparatus comprises cooperating jaws with stomach engaging surfaces which define a relatively open area which permits the passage of food at a restricted rate. The apparatus is secured in place by cooperating teeth which engage the stomach wall and by sutures formed between the stomach wall and apertures formed in the jaws.

US 2007/0021761 A1 discloses a clamp device for placating the stomach consisting of a silicon frame that is U-shaped with opposing legs self-hinged to a bight portion. The legs have inner surfaces that sealingly carry an inflatable balloon which can be inflated or aspirated to adjust the gastric restriction stoma. A flexible latch member carried by one of the legs has a serration which is inserted into a latch cavity of the opposing leg to lock the legs together. Lumens within the legs communicate with a fluid supply source and respective inflatable balloon.

WO 2005/092210 A1 discloses devices for creating one or more plications within a hollow body organ.

EP 1 749 506 A1 discloses a method for gastric reduction surfery which includes applying a clamp to the stomach to form a gastric pouch. The clamp includes a body shaped to create a gastric pouch of standard size.

### SUMMARY OF THE INVENTION

The present invention provides a restriction system for joining anterior and posterior walls of a gastric cavity in the performance of gastric reduction surgery as recited in the claims.

There is also disclosed a restriction system for joining anterior and posterior walls of a gastric cavity in the performance of the gastric reduction surgery not according to the present invention. The restriction system includes a concave first retention bar shaped and dimensioned for positioning along an anterior cavity wall and a first locking bar shaped and dimensioned for secure attachment along an opposite posterior cavity wall and placement within a retention cavity defined by the first retention bar.

The first retention bar may include a substantially planar wall extending between a first end and a second end of the retention bar, a first retention hook member extends from the first end of the planar wall and a second retention hook member extends from the second end of the planar wall, the first retention hook member and the second retention hook member being substantially C-shaped with respective concave recesses facing each other in an opposed manner defining a substantially C-shaped arrangement when the retention bar is viewed from along a cross section aligned with an axis extending between the first retention hook member and the second retention hook member.

The locking bar may be a substantially planar member shaped and dimensioned to seat within the concave space of the retention cavity as defined by the first retention bar.

The first locking bar may be flexible to compress and expand for placement and seating within the concave space of the first retention bar.

The restriction system may include a second retention bar coupled to the first retention bar, such that concave spaces of the first retention bar and the second retention bar face in an opposed manner.

The restriction system may include a second locking bar linked to the first locking bar.

There is also disclosed a method for gastric reduction surgery not according to the present invention, comprising the steps of engaging a posterior cavity wall of a gastric cavity, engaging an anterior cavity wall of the gastric cavity, and bringing the anterior cavity wall and the posterior cavity wall together to form a junction.

The step of engaging may include deploying a suture anchoring device within the posterior cavity wall at a first location.

The step of engaging may include the step of deploying a suture anchoring device within the posterior cavity wall at a second location adjacent the first location.

The step of bringing may include applying tension to suture material extending from the respective suture anchoring device at the first location and the suture anchoring device at the second location to draw the posterior cavity wall across the gastric cavity and into contact with the anterior cavity wall.

The method may include the step of locking the suture material in a tensioned state.

The step of engaging the posterior cavity wall may include grasping a portion of the posterior cavity wall and forming a fold of tissue composed of two layers of posterior cavity wall tissue.

The method may include the step of securing the fold with a suture anchoring device.

The step of bringing may include tensioning suture material of the suture anchoring device to draw the posterior cavity wall across the gastric cavity and into contact with the anterior cavity wall.

The method may include the step of locking the suture material in a tensioned state.

Other objects and advantages of the present invention will become apparent from the following detailed description when viewed in conjunction with the accompanying drawings, which set forth certain embodiments of the invention.

### BRIEF DESCRIPTION OF THE DRAWINGS

Figure 1 is a schematic view of a gastric cavity and gastroscope.
Figure 2 is a cross-sectional view of an abdominal wall and gastric cavity-showing a fastener deployed through a posterior wall.
Figure 3 is a cross-sectional view of an abdominal wall and gastric cavity showing suture material tensioned to draw anterior and posterior cavity walls together.
Figure 4 is a cross-sectional view of an abdominal wall and gastric cavity showing an abdominal wall lock.
Figure 5 is an anterior view of a gastric cavity partially broken away to show a grasper and deployment needle inside the cavity.
Figure 6 is a cross-sectional view of an abdominal wall and gastric cavity showing an abdominal wall lock created with a posterior wall tissue fold.
Figure 7 is a cross-sectional view of a gastric cavity showing an alternative embodiment for forming a posterior wall tissue fold.
Figure 8 is a longitudinal, sectional view of a gastric cavity showing a pair of posterior to anterior wall junctions.
Figure 9 is a front view of the exterior of a gastric cavity showing a plurality of posterior to anterior wall junctions formed with a circular stapler.
Figure 10 is a front view of the exterior of a gastric cavity showing a pair of posterior to anterior wall junctions connected by a bar.
Figure 11 shows an alternate embodiment of a device for connecting the anterior and posterior cavity walls of the gastric cavity.
Figure 12 is a line drawing showing the gastric cavity to be transparent.
Figure 13 is a line drawing of the gastric cavity showing, in solid lines, the two-ring restriction system in place.
Figure 14 shows the outer ring of the two-ring restriction system.
Figure 15 shows the outer ring folded for insertion.
Figure 16 shows the inner ring of the two-ring restriction system in the form of a spring ring.
Figure 17 shows the inner ring of Nitinol prior to installation in the outer ring.
Figure 18 shows the inner ring of Nitinol in a state prior to insertion into the body.

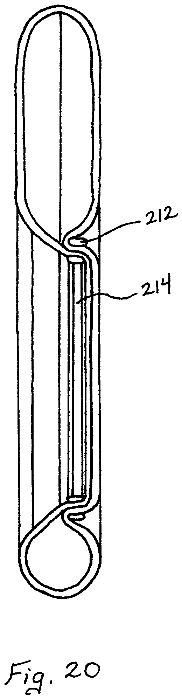

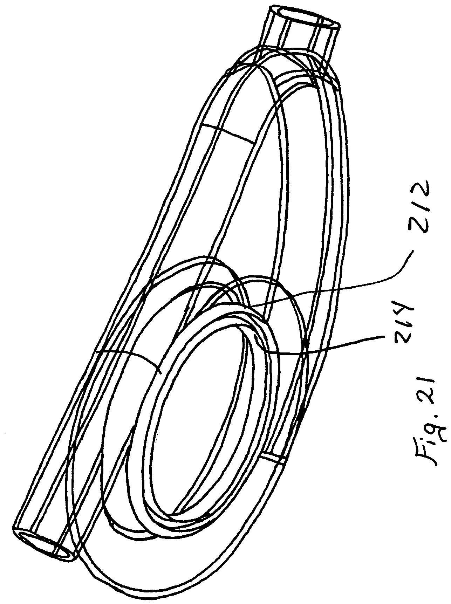

Figures 19, 20 and 21 show various views of an alternate embodiment of a device for connecting the anterior and posterior cavity walls of the gastric cavity.
Figure 22 shows an alternate embodiment of a device connecting the anterior and posterior cavity walls of the gastric cavity.
Figure 23 shows an alternate embodiment of a device for connecting the anterior and posterior cavity walls of the gastric cavity.
Figure 24 shows an alternate embodiment of a device for connecting the anterior and posterior cavity walls of the gastric cavity.
Figure 25 shows an alternate embodiment of a device for connecting the anterior and posterior cavity walls of the gastric cavity.
Figure 26 shows an alternate embodiment of a device for connecting the anterior and posterior cavity walls of the gastric cavity.

### DESCRIPTION OF THE PREFERRED EMBODIMENT

The detailed embodiments of the present invention are disclosed herein. It should be understood, however, that the disclosed embodiments are merely exemplary of the invention, which may be embodied in various forms. Therefore, the details disclosed herein are not to be interpreted as limiting, but merely as a basis for teaching one skilled in the art how to make and/or use the invention.

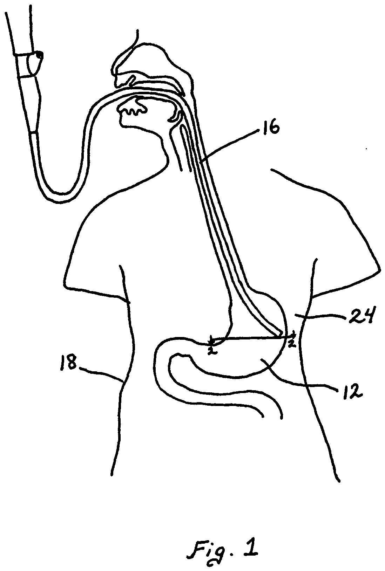

Available stomach volume is restricted, reduced, or otherwise partitioned by joining the anterior and posterior cavity walls 12, 14 at one or more locations within the gastric cavity 10. Joining the anterior and posterior cavity walls 12, 14 of the gastric cavity 10 together creates a restriction within the gastric cavity 10, which reduces the available volume for food. To join the anterior and posterior cavity walls 12, 14, a flexible gastroscope 16 is passed transesophageally into the gastric cavity 10, as shown in Figure 1. The gastroscope 16 provides insufflation, illumination and visualization of the gastric cavity 10, as well as a passageway into the gastric cavity 10. The gastric cavity 10 is insufflated through the gastroscope 16 to create a sufficiently rigid working surface that may be pierced without damaging the opposing wall of the gastric cavity 10. Access to the peritoneal cavity sufficient for gastric manipulation and/or procedures from outside of the gastric cavity can be obtained through several means including but not limited to one or more of the following: open surgical technique, laparoscopic surgical techniques involving one or more abdominal incisions (includes percutaneous access and manipulation), and natural orifice approaches (oral, anal, vaginal, etc.). Of course, gastroscope 16 or similar may also be used for endolumenal procedure steps from within the gastric cavity. Insufflation of the gastric cavity 10 may also allow the boundaries of the gastric cavity 10 to be mapped out by external palpation. The pressure on the abdominal wall 18 is observed within the gastric cavity 10 through the gastroscope 16 to determine the placement of one or more trocar(s), or other port device allowing abdominal access.

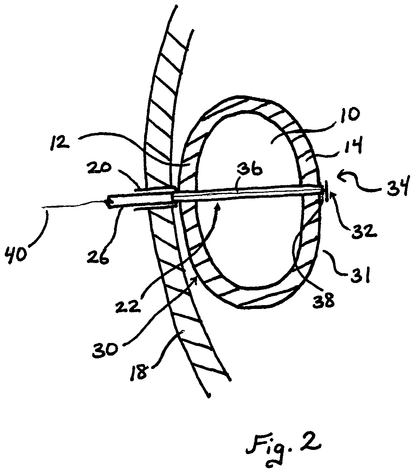

After the gastric cavity 10 has been mapped through the gastroscope 16, a trocar 20 is inserted through the abdominal wall 18. Figure 2 shows a single trocar 20 inserted through an abdominal wall 18. The trocar 20 preferably has a diameter of between approximately 3mm and approximately 5mm to allow an adequate passageway for instruments and suture anchoring devices. With the trocar 20 inserted into the abdominal wall 18, a suture anchor deployment needle 22 is passed through the trocar 20 into the abdominal cavity 24. The suture anchor deployment needle 22 includes an outer, protective sheath 26. An axial lumen (not shown) extends through the suture anchor deployment needle 22 for retaining the suture anchoring devices for release within the gastric cavity 10. Prior to inserting the suture anchor deployment needle 22, the sheathed tip 28 of the suture anchor deployment needle 22 is pressed against the exterior surface 30 of the anterior cavity wall 12 of the gastric cavity 10 to indent the wall. The cavity wall indentation is visualized through the gastroscope 16 to determine the proper location to insert the suture anchor deployment needle 22. After the proper insertion location is determined, the sheath 26 is drawn back and the tip 28 of the suture anchor deployment needle 22 is pushed into the interior of the gastric cavity 10. Once inside the gastric cavity 10, the suture anchor deployment needle 22 is advanced through the gastric cavity 10 and into and through the posterior cavity wall 14. As shown in Figure 2, a suture anchoring device 32 is deployed from the suture anchor deployment needle 22 into or through the posterior cavity wall 14. Tissue manipulation devices (graspers, snares, vacuum suction, temporary helical tissue embedding anchors, etc.) can be used to grab the posterior cavity wall 14 either endoscopically or laparoscopically and create space on the exterior surface 34 of the posterior cavity wall 14 for the suture anchor device 32 to be deployed through the posterior cavity wall 14.

In the embodiment shown in Figure 2 the suture anchoring device is a t-tag fastener 32. A length of suture material 36 is attached at a distal end 38 to the t-tag fastener 32. However, other types of tissue fasteners suitable for holding together portions of the gastric cavity wall may also be used in the present invention. Examples include t-type anchors as already discussed, reconfigurable "basket"-type anchors (which generally comprise a number of configurable struts or legs extending between two collars or support members), and linear anchors (elongate anchors which are configured to fold or become compressed into a bowed or expanded configuration). In general, anchor characteristics are such that prior to deployment they can easily be placed into or through tissue(s), but after deployment, have an altered configuration providing at least one dimension sufficiently large to prevent the anchor from being pulled back through the tissue it was deployed in.

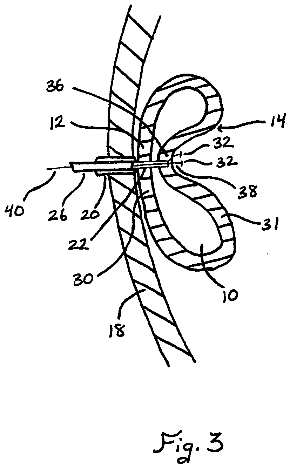

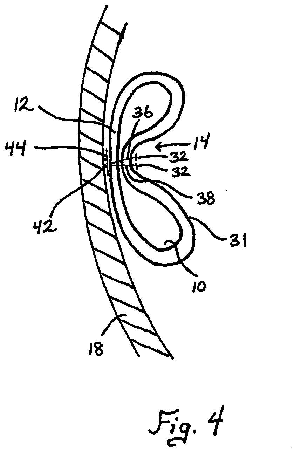

After the t-tag fastener 32 is deployed through the posterior cavity wall 14, the suture anchor deployment needle 22 is moved to a second, adjacent location along the posterior cavity wall 14. At this second location, another suture anchoring device 32 is deployed from the suture anchor deployment needle 22 into or through posterior cavity wall 14, as shown in Figure 3. The second suture anchoring device 32 is preferably also a t-tag fastener, and has a length of suture material 36 attached thereto at a distal end 38. After the first and second t-tag fasteners 32 are deployed into or through the posterior cavity wall 14, the suture anchor deployment needle 22 is withdrawn from the gastric cavity 10 and the trocar 20. As the suture anchor deployment needle 22 is withdrawn, the suture material 36 from the two deployed t-tag fasteners 32 remains within the gastric cavity 10, extending from the deployed suture anchor devices 32 through the trocar 20. Tension is applied to the proximal ends 40 of the suture material 36 to draw the posterior cavity wall 14 across the gastric cavity 10 and into contact with the anterior cavity wall 12. The anterior cavity wall 12 is in turn pulled into contact with the abdominal wall 18, as shown in Figure 3. A medical instrument may apply tension to the suture material 36 laparoscopically through the trocar 20. Alternatively, tension may be applied to the proximal ends 40 of the suture material 36 outside of the body. After the suture material 36 is tensioned to draw the anterior and posterior cavity walls 12, 14 together against the abdominal wall 18, the suture material 36 is locked in a tensioned state by applying a knotting element 42 (or other suture termination means including the application of surgical knots) to the proximal ends 40 of the suture material 36. Alternatively, the proximal ends of the suture material may be tied in a knot outside of the body. The two lengths of suture material may also be replaced with a single strand of suture material extending between the t-tag fasteners. In this embodiment, a looped length of the suture material 36 extending between the suture anchor devices 32, that is, the first and second t-tag fasteners, is drawn externally through the trocar 20, and a knotting element 42 applied to the loop to lock in the tension between the two anchoring devices 32. After applying a knotting element 42 or tying a knot, the locked suture material 36 is passed back into the abdominal cavity 24 through the trocar 20. The knot or knotting element 42 is positioned on the exterior of the gastric cavity 10 between the anterior cavity wall 12 and the abdominal wall 18. As shown in Figure 4, a buttress material 44 may be placed between the knotting element 42 and the exterior of the anterior cavity wall 12. The buttress material 44 slows or prevents the erosion of the knotting element 42 through the anterior cavity wall 12.

To encourage or promote healing between the anterior and posterior interior walls of the stomach, treatments to promote healing may be applied. These treatments include but are not limited to providing injury to the mucosal surface (thermal, mechanical, chemical, etc.), complete removal of the mucosal surface, adding therapeutic agents (medicinal, chemical, etc.) to regions to be healed, and/or the application of appropriate levels tissue stresses that may cause controlled healing (including localized tissue necrosis).

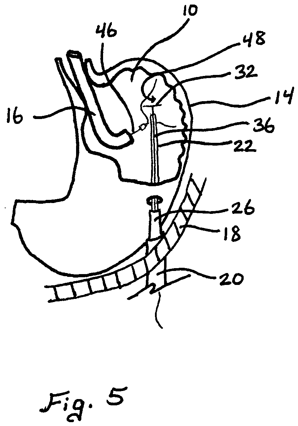

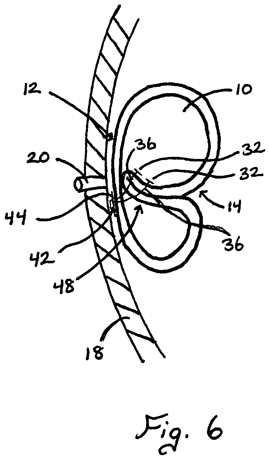

As described above, it may be necessary to create a void for the suture anchor devices 32 to enter on the exterior surface 30 of the posterior cavity wall 14 due to the close proximity of other organs. Figure 5 shows an alternative embodiment for forming a junction between the anterior and posterior cavity walls 12, 14 of a gastric cavity 10. As shown in Figure 5, a tissue grasper 46 or other tissue manipulator (graspers, snares, vacuum suction, temporary helical tissue embedding anchors, etc.) is passed into the gastric cavity 10) through the gastroscope 16. Alternately, a second transgastric instrument may be used. Within the gastric cavity 10, the grasper 46 is manipulated to grip onto a portion of the posterior cavity wall 14. With the posterior cavity wall 14 retained within the grasper 46, the grasper 46 is drawn back towards the gastroscope 16 and away from the posterior cavity wall 14. As the grasper 46 retracts away from the posterior cavity wall 14, a fold of tissue 48 composed of two layers of posterior cavity wall tissue is formed by the tissue held within the grasper 46. With the grasper 46 engaging the tissue fold 48, the suture anchor deployment needle 22 is passed through the anterior cavity wall 12 and into the interior of the gastric cavity 10. The tip 28 of the suture anchor deployment needle 22 is advanced against the tissue fold 48, and a t-tag fastener 32 is deployed from the suture anchor deployment needle 22 into or through the two layers of posterior cavity wall tissue 14 of the tissue fold 48. Multiple fasteners may be deployed in this manner to reinforce the fold. The suture material 36 is again attached at a distal end 38 to the t-tag fastener 32.
After deployment of the t-tag fastener 32, the suture anchor deployment needle 22 is moved to a second location against the tissue fold 48, and a second t-tag fastener 32 is deployed into or through the two layers of posterior cavity wall tissue of the tissue fold 48, as shown in Figure 6. The suture material 36 is attached at a distal end 38 to the second t-tag fastener 32. After the pair of t-tag fasteners 32 is deployed into the tissue fold 48 formed within the posterior cavity wall 14, the deployment needle 22 is withdrawn from the gastric cavity 10 through the anterior cavity wall 12. As the suture anchor deployment needle 22 is withdrawn, the suture material 36 remains within the gastric cavity 10, extending from deployed t-tag fasteners 32 through the anterior cavity wall 12 (potentially through multiple access points on the anterior surface). Tension is applied to the proximal ends 40 of the suture material 36 through the trocar 20 to draw the posterior cavity wall 14 across the gastric cavity 10 and into contact with the anterior cavity wall 12. After the anterior and posterior cavity walls 12, 14 are drawn together, a knotting element 42 is applied to the tensioned suture material 36 to lock the posterior cavity wall 14 against the anterior cavity wall 12, as shown in Figure 6. The knotting element 42 or knot is positioned between the anterior cavity wall 12 and the abdominal wall 18. A pre-tied slip knot may also be used to facilitate this tissue apposition particularly in, but not limited to the case where multiple access sites are used on the anterior surface of the stomach. An example of which is disclosed in further detail in commonly owned and pending U.S. Patent Publication Number US 2009-0024144 A1. As mentioned above, a buttress material 44 may be placed between the knotting element 42 and the anterior cavity wall 12 to slow or prevent erosion through the cavity wall 12.

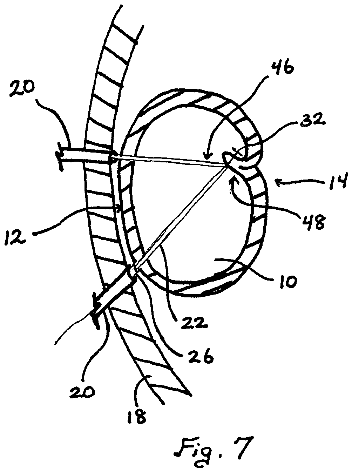

As an alternative to passing the grasper 46 through gastroscope 16, a second trocar 20 may be inserted through the abdominal wall 18 to accommodate passage of the grasper 46, as shown in Figure 7. In this embodiment, the grasper 46 engages and retains a folded portion 48 of the posterior cavity wall 14 while the t-tag deployment needle 22 is advanced into contact with the tissue. After the t-tag fasteners 32 are deployed into the posterior cavity wall 14, the deployment needle 22 and the grasper 46 are withdrawn through the trocars 20. The suture material 36 from the deployed t-tag fastener 32 is then drawn through the gastric cavity 10 and the anterior cavity wall 12. The suture material 36 is then tensioned, as described above, to draw the tissue fold 48 formed in the posterior cavity wall 14 into contact with the anterior cavity wall 12. A knot or knotting element 42 is applied to the suture material 36 through the trocar 20, in the manner described above, to lock the posterior cavity wall 14 tissue fold 48 against the anterior cavity wall 12. In the posterior cavity wall fold embodiment, a fold is formed in the posterior cavity wall for retaining one or more suture anchoring devices. Creating a fold eliminates the need to pass the deployment needle completely through the posterior cavity wall to deploy the anchoring devices, thereby greatly reducing the risk of unintentional damage to nearby organs (spleen, pancreas, aorta, etc.) when the deployment needle is advanced into the posterior cavity wall.

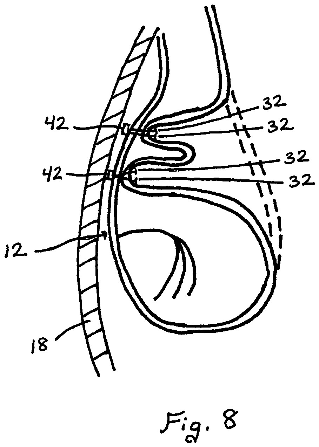

After the initial anterior cavity wall 12 to posterior cavity wall 14 junction is secured, the deployment needle 22 may again be passed through the trocar 20 and the sheathed deployment needle 22 pressed against the anterior cavity wall 12 to determine one or more additional locations for wall junctions. Additional suture anchoring devices 32 may be deployed into the posterior cavity wall 14 at additional locations, and the suture material 36 tensioned and knotted outside of the anterior cavity wall 12 to form a plurality of restrictions within the gastric cavity 10. The number of anterior to posterior restrictions created within the gastric cavit<= 10 will depend upon the desired gastric volume reduction or new stomach configuration (e.g., vertical sleeve gastroplasty, etc.). Figure 8 shows a longitudinal, sectional view of a gastric cavity 10 having a pair of wall junctions. In this example, the anterior to posterior cavity wall 12, 14 junctions are created along the length of the gastric cavity 10 between the fundus and pylorus. Aligning the anterior and posterior cavity wall 12, 14 junctions along the length of the gastric cavity 10, as shown in Figure 8, creates a vertical sleeve gastric restriction. Alternatively, a plurality of anterior to posterior wall junctions may be formed in a more random pattern to produce several "pleats" for reducing the overall stomach volume.

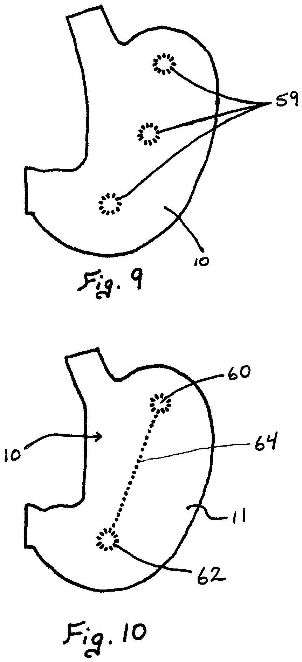

In a third embodiment of the invention as shown with reference to Figures 9 and 10, an anterior to posterior wall cavity junction is created by use of a tissue stapler. A conventional circular stapler may be used to create a junction by passing the anvil of the stapler into the abdominal cavity through a large diameter trocar or abdominal incision. Inside the abdominal cavity, the anvil is grasped by a grasping tool passed through a second, spaced trocar port. Access to the posterior surface of the stomach is created through standard dissection. Using the grasping tool, the anvil is moved adjacent to the exterior surface of the posterior cavity wall of the gastric cavity. With the aid of the removable piercing tip that is attached to anvil 54, the shaft of the anvil is passed through both the posterior and anterior cavity walls 12,14. The cartridge side of the stapler is placed adjacent the anterior cavity wall, such that the cartridge and anvil are aligned on opposite sides of the gastric cavity. The anvil is joined up through the gastric cavity with the cartridge, which is then fired to create a small hole through the anterior and posterior cavity walls that has at least one row of staples reinforcing the edges of the hole. The anterior and posterior cavity walls are joined together around the perimeter of the hole. The junction between the anterior and posterior cavity walls at the hole reduces the available capacity in the gastric cavity. After firing, the anvil and a new cartridge may be moved to a second location adjacent the exterior surfaces of the anterior and posterior cavity walls. After the anvil pierces through both walls of the stomach in the new location, the anvil again joins up with the cartridge through the anterior and posterior cavity walls. After the anvil and cartridge are joined, the cartridge is refired to create a second cavity hole.

The process of aligning the anvil and cartridge along the exterior surfaces of the anterior and posterior cavity walls, and firing the cartridge to make a hole, may be repeated numerous times to achieve the desired reduction in stomach volume. Measurements have shown that for a standard-size circular surgical stapler, the creation of 4 - 6 cavity holes can result in over a 50% reduction in overall gastric volume. The anterior and posterior cavity walls heal around the perimeter of each hole, providing effective long-term gastric volume reduction. In addition to conventional staplers, a modified surgical stapler sized to fit through a 3mm - 5mm trocar port may be used to create the holes in the gastric cavity walls. Figure 9 shows an exterior view of a gastric cavity 10 in which three holes 59 have been formed through the anterior and posterior cavity walls by use of a circular stapler. It is envisioned that multiple anastomotic devices including those used in coronary artery bypass graft surgery (e.g., Medtronic Spyder, Medtronic U-Clip, iiTech S2S Anastomotic System, Cardica C-Port, St. Jude Medical Symmetry Anastomotic System, Bypass Ltd. Corlink Proximal Anastomotic System etc.) could be appropriately scaled for use through small trocar ports to facilitate this tissue apposition.

Figure 10 is also an exterior view of a gastric cavity 10 showing a pair of circular holes 60, 62 or openings formed through the cavity walls 11. In this view, a bar 64 is attached between the circular holes 60, 62 so as to extend along the exterior surfaces of the gastric cavity 10. The bars 64 may be applied to both the anterior and posterior sides of the gastric cavity 10 to extend between the circular holes 60, 62. Adding bars 64 to the exterior of the gastric cavity 10 and tensioning the first and second hole 60, 62 away from each other creates an obstruction within the gastric cavity 10 that is similar to a tissue wall, thereby creating a partition within the gastric cavity 10 and further reducing the stomach volume. In one embodiment, the location and placement of the bars and holes may be chosen to create a tissue geometry that approximates that of a vertical sleeve gastroplasty or the Magenstrasse and Mill procedure.

In each of the above-described embodiments, two distinct areas of the inner lining or mucosa of the gastric cavity are brought together and locked into contact in order to form a permanent restriction within the gastric cavity. To enhance healing between these contacting tissues, a resection is preferably performed on the portions of the mucosa to be joined, in order to remove the innermost layer of the gastric cavity wall. This resection enhances healing and, thus, the durability of the gastric volume reduction procedure. Other means of promoting healing have been described above.

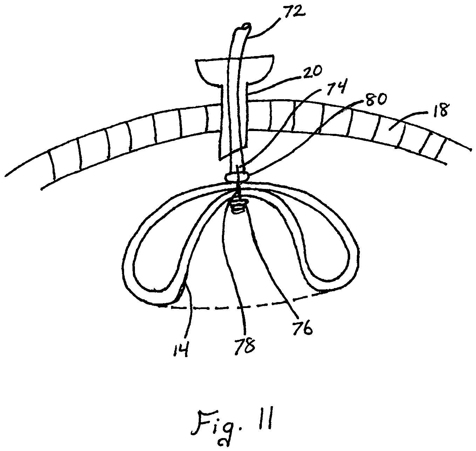

In accordance with yet another embodiment and with reference to Figure 11, a device of connecting the anterior and posterior cavity walls 12, 14 of the gastric cavity 10 together in a hybrid fashion through a small trocar port 20 for a gastric volume reduction or a restrictive sleeve procedure is disclosed. First a trocar 20 is placed in the abdominal wall 18 above the anterior surface 70 of the gastric cavity 10. The obturator is removed and a device 72 is placed in the trocar 20. This device 72 is located under visualization of the endoscope (not shown) from within the stomach. It is gently placed in the anterior surface 70 of the gastric cavity 10. A hollow needle 74 is then passed through the anterior cavity wall 12 of the gastric cavity 10. This needle 74 passes through to the posterior cavity wall 14 and a piercing tissue grasping device such as a helical corkscrew needle 76 extends out the tip 78 of the hollow needle 74. The corkscrew needle 76 is advanced into the tissue of the posterior cavity wall 14 and safely lifts the posterior cavity wall 14 up in connection with the anterior cavity wall 12. Again it is preferable that the interior surfaces of the stomach to be approximate are treated to promote healing as previously described. The previously treated mucosal walls of the tissue are brought together on the anterior surface 70 where a fastener such as a box staple 80 fires from the outside surface of the gastric cavity 10, firing into the helical corkscrew needle 76. Once the box staple 80 and the helical corkscrew needle 76 are connected with the two opposing walls 12, 14 of the gastric cavity 10 between, volume reduction has been achieved. All is safely accomplished under direct visualization from within the gastric cavity 10. The backspan of box staple 80 may be reinforced with a buttress to distribute the tissue loads more evenly over a wider area.

The previously treated mucosal walls of tissue are preferably treated with any number of standard EMR (endoscopic mucosal resection) treatments. Endoscopic mucosal resection is an endoscopic procedure that is now used most often to remove an area of high-grade dysplasia or small, early cancer. The FDA has approved two devices for EMR; the Olympus EMR cap and the Wilson-Cook Duette. EMR might also be used in the future to increase durability of Endo-luminal procedures and precise control of EMR location would be critical. U.S. Patent Nos. 7,186,252, entitled, "Endoscopic Mucosal Resection Device and Method of Use", 7,169,115, entitled "Endoscopic Mucosal Resection Device with Overtube and Method of Use", and 6,994,705, entitled, "Endoscopic Mucosal Resection Device with Conductive Tissue Stop".

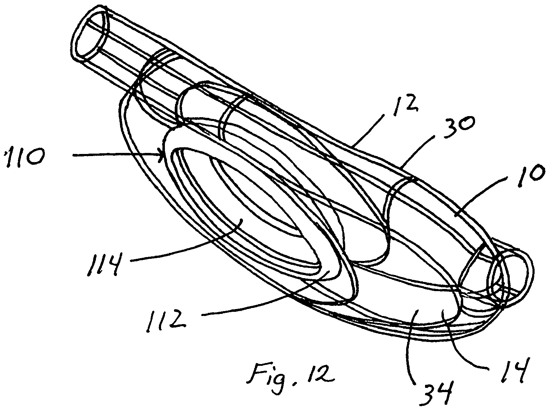

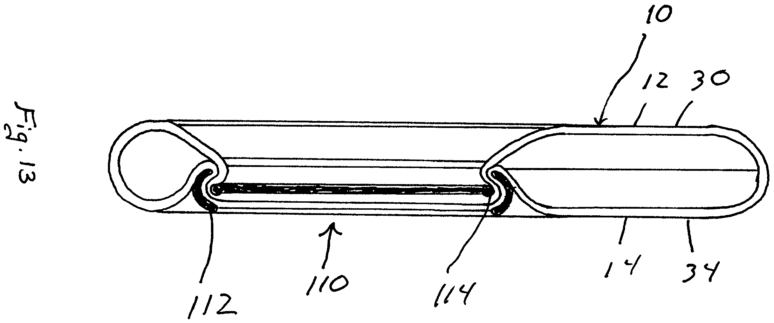

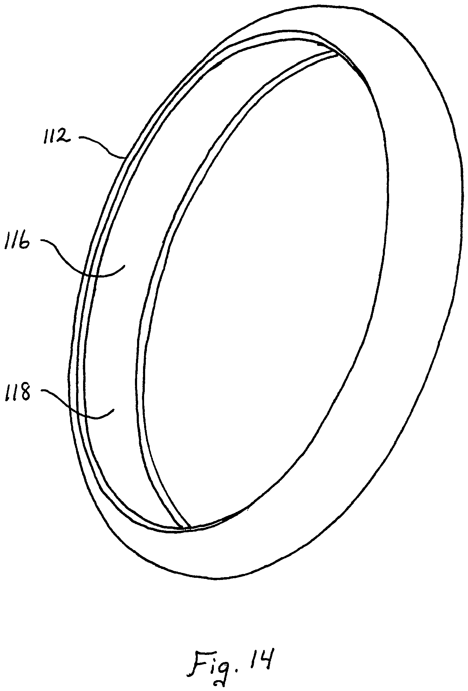

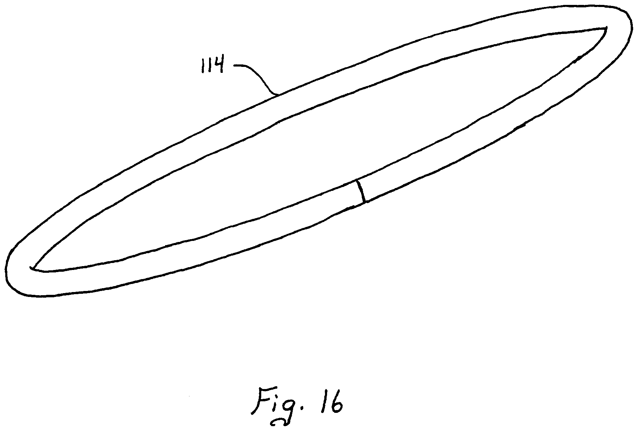

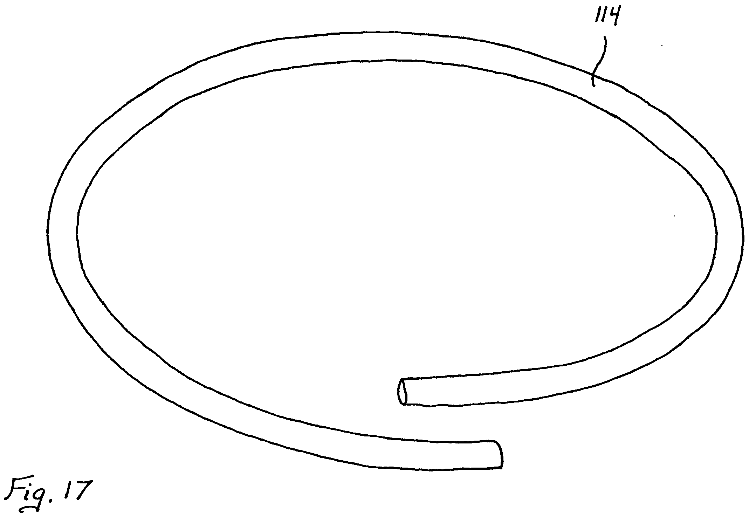

With references to Figures 12 to 18, an embodiment for restricting stomach volume is disclosed. In accordance with this embodiment, a two-ring restriction system 110 is disclosed. The restriction system 110 generally includes a first restriction ring 112 and second restriction ring 114 shaped and dimensioned to seat within the first restriction ring 112 for effectively decreasing the volume available within the gastric cavity 10. As such, the first restriction ring 112 has a larger diameter than the second restriction ring 114, along the second restriction ring 114 to seat along the inner surface 116 of the first restriction ring 112 in a manner discussed below in greater detail. Briefly a first restriction ring 112 is positioned along the exterior surface 30, 34 along either the anterior cavity wall 12 or the posterior cavity wall 14 (in accordance with the present disclosure it is positioned along the posterior cavity wall, although this could be altered without departing from the scope of the present invention). Thereafter, a second restriction ring 114 is positioned along the exterior surface 30 of the gastric cavity 10 opposite the first restriction ring 112. The first and second restriction rings 112, 114 are then forced toward each other entrapping and folding tissue therebetween. The first restriction ring 112 is then positioned so as to compress and force the second restriction ring 114 into contact with the inner surface 116 of the first restriction ring 112 with stomach tissue positioned therebetween. The retaining force between the first restriction ring 112 and the second restriction ring 114 maintains all of the components (including the tissue locked therebetween) in a static arrangement producing a reduction in stomach volume.

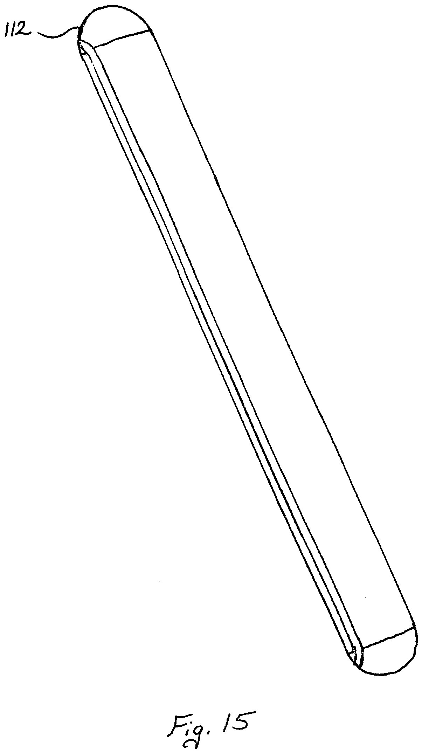

In accordance with a preferred embodiment, the first restriction ring 112 is substantially circular and is composed of a resilient material having a C-shaped cross section when viewed along a plane transverse to the plane in which the first restriction ring 112 lies. The first restriction ring 112 is sufficiently flexible to permit it to be compressed as shown in Figure 15 for insertion and deployment. As will be appreciated based upon the foregoing disclosure, the second restriction ring 114 is shaped to seat within a concave portion, that is, the seating recess 118 along the inner surface 116 of the first restriction ruing 112.

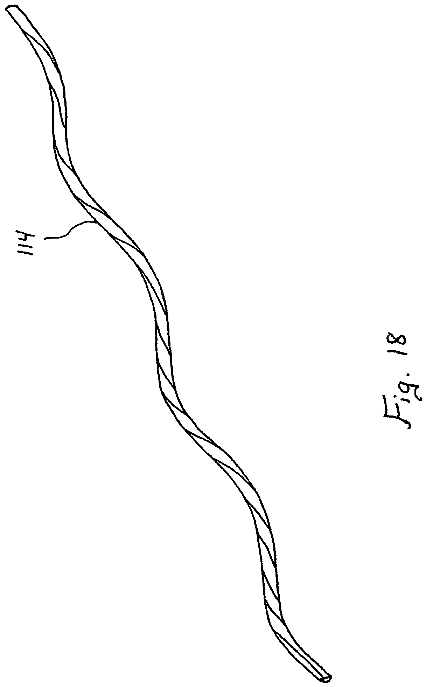

Referring to the second restriction ring 114, it is a circular member also composed of a flexible material. When fully deployed as shown with reference to Figures 12, 13, 16 and 17, the second restriction ring 114 is a substantially round member which ultimately takes a circular configuration when deployed in accordance with the present invention. The second restriction ring 114, when fully deployed and expanded, has a substantially circular cross section when viewed along a plane transverse to the plane in which the second restriction ring 114 lies. As such, the second restriction ring 114 is shaped and dimensioned for selective seating within the seating recess 118 formed along the inner surface 116 of the first restriction ring 112. Considering that the second restriction ring 114 must be compressed to a low profile (see Figure 18) for deployment within the abdominal cavity 24, the second restriction ring 114 is composed of a shape memory material, for example Nitinol, or a spring material, which upon deployment will reorient from its insertion configuration (see Figure 18) to its circular deployed configuration (see Figures 12, 13, 16 and 17). As such, the second restriction ring 114 may be compressed, passed through a delivery device and deployed within the abdominal cavity 24 for positioning within the seating recess 118 formed along the inner surface 116 of the first restriction ring 112.

In practice, the first restriction ring 112 is laparoscopically deployed within the abdominal cavity 24 at a location along either the anterior or posterior cavity wall 12, 14 of the gastric cavity 10. Thereafter, the second restriction ring 114 is similarly deployed within the abdominal cavity 24 along the opposite cavity wall 12, 14 of the gastric cavity 10. The second restriction ring 114 is then pressed against the exterior surface 30, 34 of the cavity wall 12, 14 toward the first restriction ring 112 and, as such, compresses the gastric cavity 10. The second restriction ring 114 continues to be forced toward the first restriction ring 112 such that the second restriction ring 114 fits within the first restriction ring 112 and seats within the concave seating recess 118 along the inner surface 116 of the first restriction ring 112 defined by the C-shaped cross section of the first restriction ring 112. When such an arrangement is achieved, the seating recess 118 of the first restriction ring 112 holds two layers of the gastric cavity 10 (that is, the anterior and posterior cavity walls 12, 14 firmly between the second restriction ring 114 and the first restriction ring 112. As such, the area inside the first restriction ring 112 and the second restriction ring 114 is prevented from collecting food and, therefore, the volume capacity of the gastric cavity 10 is reduced.

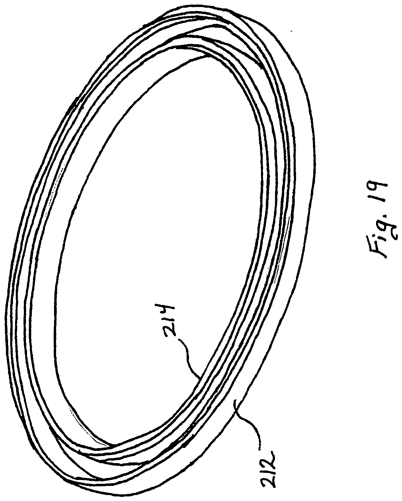

In accordance with an alternate embodiment, and with reference to Figures 19 20 and 21, the first and second restriction rings 212, 214 may be composed of magnetic materials which are drawn together such that the first and second restriction rings 212, 214 are retained in an aligned orientation holding tissue in a manner similar to that disclosed with reference to Figures 12 to 18.

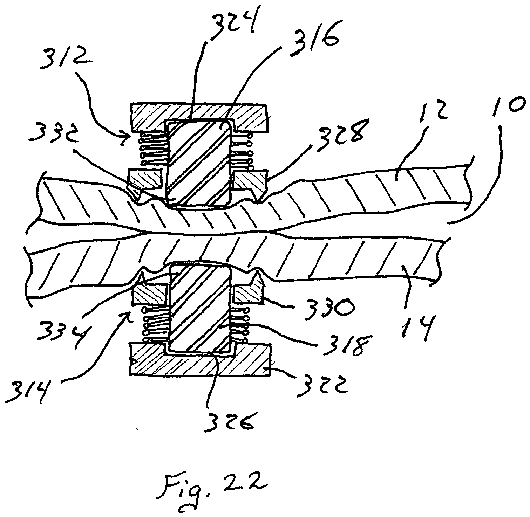

In accordance with yet a further embodiment, and with reference to Figure 22, spring loaded flanges are positioned on opposite sides of the gastric cavity 10 so as to draw the anterior cavity wall 12 and the posterior cavity wall 14 together. More particularly, each of the first and second restriction rings 312, 314 are composed of an annular shaped central magnetic member 316, 318 having a spring retention flange 320, 322 secured to a first end 324, 326 thereof and a spring loaded flange 328, 330 mounted for movement relative to the second end 332, 334 thereof. As such, and when a pair of spring loaded flanges 328, 330 in a circular orientation are held along opposite cavity walls 12, 14 of the gastric cavity 10, the annular shaped central magnetic members 316, 318 are drawn together while the spring retention flanges 320, 322 maintain the spring loaded flange 328, 330 in a loaded manner forcing the anterior cavity wall 12 toward the posterior cavity wall 14.

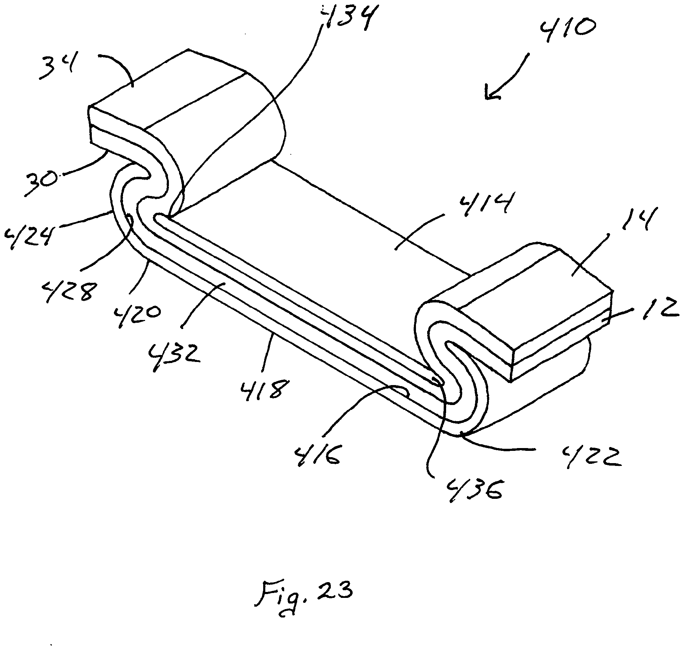

In accordance with yet another embodiment, and with reference to Figure 23, a beam and bar system 410 is utilized. In accordance with this embodiment, a concave outer retention bar 412 is positioned along the anterior cavity wall 12 while a locking bar 414 is secured along the opposite posterior cavity wall 14 and, ultimately within the retention cavity 416 defined by the retention bar 412. In accordance with the present disclosure the retention bar 412 is positioned along the anterior cavity wall 12.

The retention bar 412 includes a substantially planar wall 418 extending between a first end 420 and a second end 422 of the retention bar 412. The retention bar 412 is of a predetermined depth sufficient to restriction stomach volume along the length of the gastric cavity 10 as will be appreciated based upon the following disclosure. A first retention hook member 424 extends from the first end 420 of the planar wall 418 and a similar second retention hook member 426 extends from the second end 422 of the planar wall 418. The first retention hook member 424 and the second retention hook member 426 are substantially C-shaped with their respective concave recesses 428, 430 facing each other in an opposed manner defining a substantially C-shaped arrangement when the retention bar 412 is viewed from along a cross section aligned with the axis extending between the first retention hook member 424 and the second retention hook member 426.

The inner, locking bar 414 is a substantially planar member shaped and dimensioned to seat within the concave space 432 of the retention cavity 416 as defined by the retention bar 412. As such, the locking bar 414 is also sufficiently flexible to compress and expand for placement and seating within the concave space 432 of the retention bar 412. As with the retention bar 412, the locking bar 414 includes a first end 434 and a second end 436, as well as a depth sufficient to restrict stomach volume or creating preferred and restricted pathways for food along the length of the gastric cavity 10 as will be appreciated based upon the following disclosure.

In particular, and in practice, the retention bar 412 is positioned adjacent the exterior surface 30 of the anterior cavity wall 12 with the concave space 432 of the retention bar 412 facing the exterior surface 30 of the cavity wall 12. Thereafter, the locking bar 414, which is positioned along an exterior surface 34 of the gastric cavity 10 opposite the exterior surface 30 of the gastric cavity 10 along which the retention bar 412 is positioned, is forced toward the retention bar 412 along the opposite cavity wall 12. As the locking bar 414 is forced toward the outer retention bar 412, the anterior cavity wall 12 and the posterior cavity wall 14 are drawn together until such a time that the first end 434 of the locking bar 414 seats within the first retention hook member 424 and the second end 436 of the locking bar 414 seats within the second retention hook member 426 retaining the tissue of the gastric cavity 10 between the retention bar 412 and the locking bar 414 in a controlled manner.

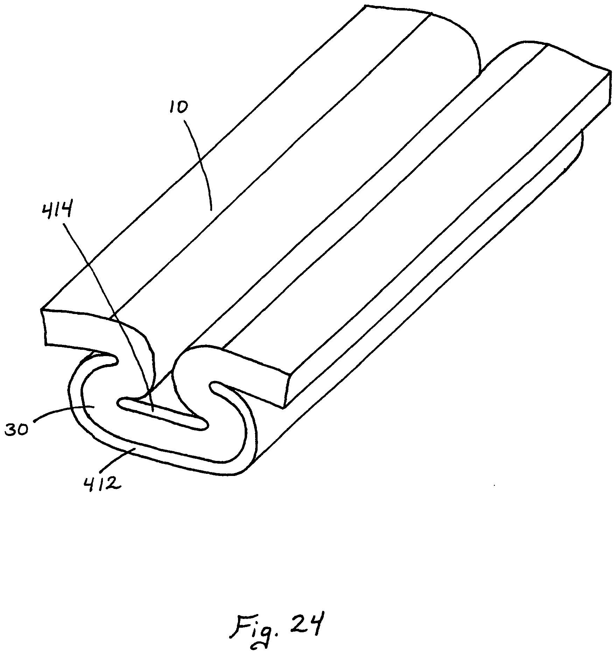

In accordance with yet an alternate embodiment, and with reference to Figure 24, the retention bar 412 and locking bar 414 may be utilized to create a fold along the internal surface of the gastric cavity 10 for reducing stomach volume. Utilizing such an embodiment, the retention bar 412 is positioned along the exterior surface 30 of the gastric cavity 10 while the locking bar 414 is positioned within the gastric cavity 10. Thereafter, a single layer of stomach tissue is forced between the retention bar 412 and the locking bar 414 in a manner folding the cavity wall 12, 14 and reducing volume of the gastric cavity 10.

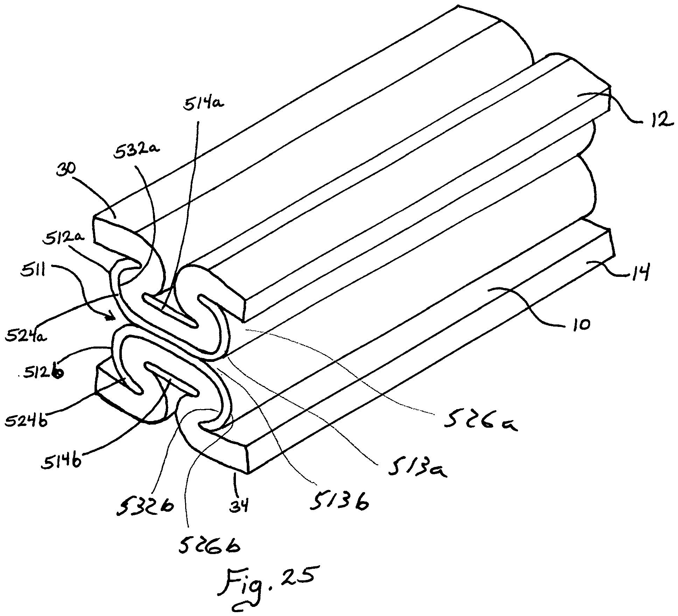

In accordance with yet a further embodiment, and with reference to Figure 25, a dual retention bar 511 is secured along the interior surface of the gastric cavity 10 such that the concave spaces 532a, 532b thereof are facing in opposite directions and locking bars 514a, 514b are secured along the exterior surface 30, 34 of the gastric cavity 10 to further reduce the stomach volume. In accordance with such an embodiment, the dual retention bar 511 is composed of first and second retention bars 512a, 512b as discussed above which are coupled along their convex surfaces 513a, 513b such that the concave spaces 532a, 532b face in an opposed manner. It is envisioned that these bars may be positioned to approximate the tissue geometry of a vertical sleeve gastroplasty or Magenstrasse and Mill procedure.

In particular, and in practice, the dual retention bar 511 is positioned within the interior of the gastric cavity 10 with the outwardly facing concave spaces 532a, 532b thereof facing the interior surface of the gastric cavity 10. Thereafter, first and second locking bars 514a, 514b, which are positioned along an exterior surface 30, 34 of the gastric cavity 10 opposite the exterior surface 30, 34 of the gastric cavity 10 along which the concave spaces 532a, 532b of the dual retention bar 511 are positioned, are forced toward the dual retention bar 511 along the opposite cavity walls 12, 14. As the locking bars 514a, 514b are forced toward the dual retention bar 511, the opposed cavity walls 12, 14 are respectively forced within the concave spaces 532a, 532b of the dual retention bar 511 until such a time that the first ends 534a, 534b of the respective locking bars 514a, 514b seat within the first retention hook members 524a, 524b and the second ends 536a, 536b of the locking bars 514a, 514b seat within the second retention hook members 526a, 526b retaining a single layer of tissue of the gastric cavity 10 between the respective first and second retention bars 512a, 512b and the first and second locking bars 514a, 514b in a controlled manner.

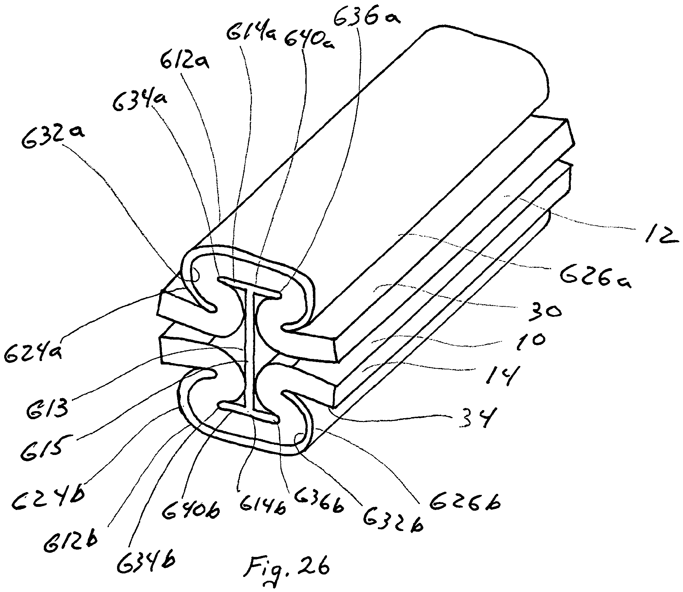

Similarly, and with reference to Figure 26, first and second retention bars 612a, 612b may be secured along the exterior surface 30, 34 of the gastric cavity 10 and a dual locking bar 613 is positioned within the gastric cavity 10 for controlling the positioning of the anterior and posterior cavity walls 12, 14 of the gastric cavity 10. The dual locking bar 613 includes first and second locking bars 614a, 614b connected by a linking member 615.

In particular, and in practice, the dual locking bar 613 is positioned within the interior of the gastric cavity 10 with the outwardly facing surfaces 640a, 640b thereof facing the interior surface of the gastric cavity 10. Thereafter, first and second retention bars 612a, 612b, which are positioned along an exterior surface 30, 34 of the gastric cavity 10 opposite the exterior surface 30, 34 of the gastric cavity 10 along which the outwardly facing surfaces 640a, 640b of the dual locking bar 613 are positioned, are forced toward the dual locking bar 613 along the opposite cavity walls 12, 14. As the dual locking bar 613 is forced toward the respective first and second retention bars 612a, 612b, the opposed cavity walls 12, 14 are respectively forced within the concave spaces 632a, 632b of the retention bars 612a, 612b until such a time that the first ends 634a, 634b of the respective locking bars 614a, 614b seat within the first retention hook members 624a, 624b and the second ends 636a, 636b of the locking bars 614a, 614b seat within the second retention hook members 626a, 626b retaining a single layer of tissue of the gastric cavity 10 between the respective first and second retention bars 612a, 612b and the first and second locking bars 614a, 614b in a controlled manner.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used system is obtained and if necessary cleaned. The system can then be sterilized. In one sterilization technique, the system is placed in a closed and sealed container, such as a plastic or TYVEK bag. The container and system are then placed in a field of radiation that can penetrate the container, such as gamma radiation, x-rays, or high-energy electrons. The radiation kills bacteria on the system and in the container. The sterilized system can then be stored in the sterile container. The sealed container keeps the system sterile until it is opened in the medical facility.

It is preferred that the device is sterilized. This can be done by any number of ways known to those skilled in the art including beta or gamma radiation, ethylene oxide, and steam.

While the preferred embodiments have been shown and described, it will be understood that there is no intent to limit the invention by such disclosure, but rather, is intended to cover all modifications and alternate constructions falling within the scope of the invention as defined by the claims.

## Claims

1. A restriction system (110) for joining anterior and posterior walls of a gastric cavity in the performance of gastric reduction surgery, comprising:
a first restriction ring (112);
a second restriction ring (114) shaped and dimensioned for engagement with the first restriction ring (112) for effectively decreasing volume available within the gastric cavity, **characterised in that**:
the second restriction ring (114) is composed of a flexible material, wherein
the second restriction ring (114) is composed of a shape memory material, which upon deployment reorients from an insertion configuration to a circular deployed configuration, and
the first restriction ring (112) has a larger diameter than the second restriction ring (114), such that the second restriction ring (114) is selectively seated along an inner surface of the first restriction ring (112).

2. The restriction system (110) according to claim 1, wherein the first restriction ring (112) is substantially circular and is composed of a resilient material having a C-shaped cross section when viewed along a plane transverse to the plane in which the first restriction ring (112) lies.

3. The restriction system (110) according to claim 2, wherein the first restriction ring (112) is flexible to permit it to be compressed for insertion and deployment.

4. The restriction system (110) according to claim 2, wherein the second restriction ring (114) is shaped to seat within a seating recess (118) along the inner surface (116) of the first restriction ring (112).

5. The restriction system (110) according to claim 1, wherein the first restriction ring (112) is composed of a magnetic material and the second restriction ring (114) is composed of a magnetic material, the first restriction ring (112) and the second restriction ring (114) being selectively drawn together such that the first and second restriction rings (112, 114) are retained in an aligned orientation holding tissue therebetween.

6. The restriction system (110) according to claim 5, wherein the first restriction ring (112) includes a spring retention flange (320) and a spring loaded flange (328) mounted for movement relative thereto and the second restriction ring (114) includes a spring retention flange (322) and a spring loaded flange (330) mounted for movement relative thereto.

## Patentansprüche

1. Restriktionssystem (110) zur Verbindung der Vorder- und Hinterwand einer Magenhöhle bei der Durchführung einer Magenreduktionsoperation, das Folgendes umfasst:
einen ersten Restriktionsring (112);
einen zweiten Restriktionsring (114), der so geformt ist und solche Abmessungen aufweist, dass er mit dem ersten Restriktionsring (112) in Eingriff gebracht werden kann, um das in der Magenhöhle vorhandene Volumen wirksam zu verkleinern, **dadurch gekennzeichnet, dass**:
der zweite Restriktionsring (114) aus einem flexiblen Material besteht, worin der zweite Restriktionsring (114) aus einem Formgedächtnismaterial besteht, das bei Ablage seine Orientierung von einer Einführungskonfiguration in eine kreisförmige abgelegte Konfiguration ändert; und
der erste Restriktionsring (112) einen größeren Durchmesser aufweist als der zweite Restriktionsring (114), so dass der zweite Restriktionsring (114) selektiv auf einer Innenfläche des ersten Restriktionsrings (1.12) angeordnet wird.

2. Restriktionssystem (110) nach Anspruch 1, worin der erste Restriktionsring (112) im Wesentlichen kreisförmig ist und aus einem nachgiebigen Material mit einem C-förmigen Querschnitt bei Betrachtung entlang einer Ebene, die quer zu der Ebene liegt, in welcher der erste Restriktionsring (112) liegt, besteht.

3. Restriktionssystem (110) nach Anspruch 2, worin der erste Restriktionsring (112) flexibel ist, damit er zur Einführung und Ablage zusammengedrüclct werden kann.

4. Restriktionssystem (110) nach Anspruch 2, worin der zweite Restriktionsring (114) so geformt ist, dass er in einer Sitzvertiefung (118) auf der Innenfläche (116) des ersten Restriktionsrings (112) sitzen kann.

5. Restriktionssystem (110) nach Anspruch 1, worin der erste Restriktionsring (112) aus einem magnetischen Material besteht und der zweite Restriktionsring (114) aus einem magnetischen Material besteht, wobei der erste Restriktionsring (112) und der zweite Restrilctionsring (114) selektiv zueinander gezogen werden, so dass die ersten und zweiten Restriktionsringe (112, 114) in einer fluchtenden Orientierung gehalten werden und Gewebe zwischen sich festhalten.

6. Restriktionssystem (110) nach Anspruch 5, worin der erste Restriktionsring (112) einen Federhalteflansch (320) und einen federbelasteten Flansch (328) für eine Relativbewegung aufweist und der zweite Restriktionsring (114) einen Federhalteflansch (322) und einen federbelasteten Flansch (330) für eine Relativbewegung aufweist.

## Revendications

1. Système d'étranglement (110) pour joindre des parois antérieure et postérieure d'une cavité gastrique lors de l'exécution d'une chirurgie de réduction de volume gastrique, comprenant:
un premier anneau d'étranglement (112);
un deuxième anneau d'étranglement (114) qui est configuré et dimensionné de manière à s'engager avec le premier anneau d'étranglement (112) afin de diminuer efficacement le volume disponible à l'intérieur de la cavité gastrique, **caractérisé en ce que**:
le deuxième anneau d'étranglement (114) est composé d'un matériau flexible, dans lequel:
le deuxième anneau d'étranglement (114) est composé d'un matériau à mémoire de forme qui, lors du déploiement, se ré-oriente d'une configuration d'insertion vers une configuration déployée circulaire; et
le diamètre du premier anneau d'étranglement (112) est supérieur à celui du deuxième anneau d'étranglement (114), de telle sorte que le deuxième anneau d'étranglement (114) soit logé de façon sélective le long d'une surface intérieure du premier anneau d'étranglement (112).

2. Système d'étranglement (110) selon la revendication 1, dans lequel le premier anneau d'étranglement (112) est sensiblement circulaire et est composé d'un matériau élastique qui présente une section transversale en forme de C lorsque l'on regarde le long d'un plan transversal au plan dans lequel le premier anneau d'étranglement (112) se trouve.

3. Système d'étranglement (110) selon la revendication 2, dans lequel le premier anneau d'étranglement (112) est flexible de manière à lui permettre d'être comprimé en vue de son insertion et de son déploiement.

4. Système d'étranglement (110) selon la revendication 2, dans lequel le deuxième anneau d'étranglement (114) est configuré de manière à se loger à l'intérieur d'un évidement de réception (118) le long de la surface intérieure (116) du premier anneau d'étranglement (7.12).

5. Système d'étranglement (110) selon la revendication 1, dans lequel le premier anneau d'étranglement (112) est composé d'un matériau magnétique, et le deuxième anneau d'étranglement (114) est composé d'un matériau magnétique, le premier anneau d'étranglement (112) et le deuxième anneau d'étranglement (114) étant tirés sélectivement ensemble de telle sorte que les premier et deuxième anneaux d'étranglement (112, 114) soient retenus dans une orientation alignée en maintenant le tissu entre eux.

6. Système d'étranglement (110) selon la revendication 5, dans lequel le premier anneau d'étranglement (112) comprend une bride de retenue à ressort (320) et une bride de retenue chargée par ressort (328) qui est montée de façon à se déplacer par rapport à celle-ci, et le deuxième anneau d'étranglement (114) comprend une bride de retenue à ressort (322) et une bride chargée par ressort (330) qui est montée de manière à se déplacer par rapport à celle-ci.
